# EUROPEAN PATENT APPLICATION

(11) **EP 4 686 415 A1**
(43) Date of publication of application: **04.02.2026**
(21) Application number: 25192985.7
(22) Date of filing: 31.07.2025
(51) Int. Cl.: A23K 10/18, A23K 20/142, A23K 20/158, A23K 20/163, A23K 50/40

(54) **PET FOOD SUPPLEMENT COMPOSITION**

(30) Priority: 31.07.2024 EP 24191985
(71) Applicant: Omni Pet Ltd., London W1W 7LT (GB)
(72) Inventor: SANDELOWSKY, Guy Alain, LONDON, W1W (GB); SIVAKUMAR, Sivasankar, London, W1W (GB)
(74) Representative: Evans, Sophie Elizabeth

(57) **Abstract**

The present invention relates to a pet food supplement composition comprising: a source of resistant starch; a source of beta glucan; a probiotic; L-carnitine; and alpha lipoic acid. The pet food supplement composition can be used to promote weight loss or in the treatment of obesity.

## Description

### Field of the invention

The present invention relates to a pet food supplement composition comprising: a source of resistant starch; a source of beta glucan; a probiotic; L-carnitine; and alpha lipoic acid. The pet food supplement composition can be used to promote weight loss or in the treatment of obesity.

### Background

Being overweight or obese can have a significant impact on the health and overall quality of life of a companion animal. Obesity, for example, can lead to a reduction in life expectancy, as well as an increased likelihood of a companion animal developing additional health complications.

Techniques for weight loss include the introduction of a specific diet, portion control and reduced meal frequency. It is not as simple, however, as reducing food intake as this can lead to a deficiency in key nutrients leading to further complications. Issues can also arise through a lack of compliance with a weight loss regime proposed by a veterinary professional.

There is an unmet need for an effective weight loss regime for use in promoting weight loss or treating obesity.

### Summary of the invention

The inventors have developed a pet food supplement composition that can be used to reduce the weight of a companion animal. The weight reduction achieved improves the overall welfare of the companion animal, improving their ability to exercise and reducing the risk of additional health complications arising.

The invention provides a pet food supplement composition comprising: a source of resistant starch; a source of beta glucan; a probiotic; L-carnitine; and alpha lipoic acid; wherein the source of resistant starch is a source of resistant starch derived from oat, rice, grains, legumes, fruit, raw potato starch, or high corn resistant starch, or combinations thereof.

The invention also provides a pet food supplement composition for use in the enhancement of pet health, wherein: the pet food supplement composition is as defined herein; and the enhancement of pet health is the treatment of obesity.

Also provided is the use of a pet food supplement composition for promoting weight loss or maintaining a healthy weight, wherein the pet food supplement composition is as defined herein.

### Brief description of the figures

Figure 1 - Manufacturing flowchart for production of the pet food supplement composition as a powder.
Figure 2 - Manufacturing flowchart for production of a soft chew comprising the pet food supplement composition.

### Detailed description

### Pet food supplement composition

As described above, the pet food supplement composition comprises: a source of resistant starch; a source of beta glucan; a probiotic; L-carnitine; and alpha lipoic acid; wherein the source of resistant starch is a source of resistant starch derived from oat, rice, grains, legumes, fruit, raw potato starch, or high corn resistant starch, or combinations thereof.

The term "pet" as used herein refers to a companion animal such as a dog or a cat. Preferably the companion animal is a dog.

The term "pet food supplement composition" as used herein refers to a composition for ingestion by a pet. The pet food supplement composition is for consumption as a meal topper or at/around the same time as a meal and is typically provided as part of a nutritionally balance meal. Preferably, the pet food supplement composition described herein is designed such that a pet consuming the pet food supplement composition as part of a meal eats a nutritionally balanced meal that provides the pet with all the essential nutrients needed for good health, while also allowing the pet to reduce their weight in a controlled way.

The ingredients in the pet food supplement composition should be non-toxic for the companion animal, e.g., non-toxic for dogs.

As the skilled person knows, "resistant starch" is a type of starch that is resistant to digestion in the small intestine, meaning that the resistant starch does not release glucose in the small intestine. The resistant starch may ferment in the large intestine, although fermentability can vary with the source of resistant starch.

Starch can alternatively be classified as rapidly digestible starch or slowly digestible starch. Rapidly digestible starch and slowly digestible starch are starches that are digested in the small intestine.

The resistant starch may act as a carrier of food. When the resistant starch acts as a carrier of food the resistant starch may, for example, reduce the amount of food absorbed into the body of the companion animal, e.g., by coating food consumed as part of the diet of the companion animal.

The resistant starch may be chemically modified or unmodified resistant starch. Typically, the resistant starch is chemically unmodified.

Consumption of resistant starch has been linked with health benefits in humans. Beneficial effects include, but are not limited to, lowering blood glucose, lowering cholesterol, lowering body fat and improving mineral absorption.

Foods that are particularly high in resistant starch include rice, potatoes, whole grains (such as barley and oats), beans and legumes (such as pinto beans, black beans, soybeans, garden peas, and fava beans), plantains, and green bananas. Yellow or ripe bananas are not typically a source of resistant starch.

The pet food supplement composition usually comprises at least about 20 wt%, for example at least about 30 wt%, preferably at least about 40 wt% of the source of resistant starch based on the total weight of the pet food supplement composition.

The pet food supplement composition usually comprises up to about 90 wt%, for example up to about 70 wt%, preferably up to about 60 wt% of the source of resistant starch based on the total weight of the pet food supplement composition.

The pet food supplement composition typically comprises from about 20 wt% to about 90 wt%, for example from about 30 wt% to about 70 wt%, preferably from about 40 wt% to about 60 wt% of the source of resistant starch based on the total weight of the pet food supplement composition.

For example, the pet food supplement composition may comprise about 50 wt% of the source of resistant starch based on the total weight of the pet food supplement composition.

The source of resistant starch is derived from oat, rice, grains, legumes, fruit, raw potato starch, or high corn resistant starch, or combinations thereof. Thus, the pet food supplement composition comprises at least one source of resistant starch, for example, one, two, or three sources of resistant starch, e.g., one or two sources of resistant starch.

The source of resistant starch typically comprises green banana, for example, green banana fruit pulp, green banana skin flour, green banana fibre, green banana skin or mixtures thereof. Preferably, the source of resistant starch comprises or consists of green banana fruit pulp extract.

Alternatively, the source of resistant starch may comprise rice bran, rice fibre, dodamssal rice (including white and brown rice varieties) or mixtures thereof. For example, the pet food supplement composition may comprise rice bran.

The source of resistant starch may comprise (a) green banana, for example, green banana fruit pulp, green banana skin flour, green banana fibre, green banana skin or mixtures thereof, and (b) rice bran, rice fibre, dodamssal rice (including white and brown rice varieties) or mixtures thereof. For example, the source of resistant starch may comprise or consist of green banana fruit pulp extract and rice bran.

Preferably, the pet food supplement composition comprises from about 20 wt% to about 90 wt%, for example from about 30 wt% to about 70 wt%, preferably from about 40 wt% to about 60 wt% of the source of resistant starch based on the total weight of the pet food supplement composition, and the source of resistant starch is green banana fruit pulp extract.

The pet food supplement composition usually comprises at least about 2 wt%, for example at least about 4 wt%, preferably at least about 5 wt% of the source of beta glucan based on the total weight of the pet food supplement composition.

Beta glucans (alternatively referred to as β-glucans) are soluble fibres found, for example, in the cell walls of cereals, yeast, fungi, and bacteria. Beta-glucan is a non-starch soluble polysaccharide. The health benefits achieved by adding a source of beta glucan to the diet of a companion animal include, but are not limited to, a reduction in the risk of heart disease and/or a lowering of cholesterol levels.

Typically, the pet food supplement composition comprises up to about 30 wt%, for example up to about 20 wt%, preferably up to about 12 wt% of the source of beta glucan based on the total weight of the pet food supplement composition.

The pet food supplement composition usually comprises from about 2 wt% to about 30 wt%, for example from about 4 wt% to about 20 wt%, preferably from about 5 wt% to about 12 wt% of the source of beta glucan based on the total weight of the pet food supplement composition.

For example, the pet food supplement composition may comprise about 6.7 wt% of the source of beta glucan based on the total weight of the pet food supplement composition.

The source of beta glucan typically comprises oat beta glucan, barley beta glucan, yeast beta glucan, fungi beta glucan or mixtures thereof. Preferably, the source of beta glucan comprises or consists of oat beta glucan.

Preferably, the pet food supplement composition comprises from about 2 wt% to about 30 wt%, for example from about 4 wt% to about 20 wt%, preferably from about 5 wt% to about 12 wt% of the source of beta glucan based on the total weight of the pet food supplement composition, and the source of beta glucan is oat beta glucan.

The pet food supplement composition comprises alpha lipoic acid. Without wishing to be bound by theory, it is thought that the alpha lipoic acid may be beneficial in supressing AMPK activity, reducing appetite and increasing metabolic rate.

The pet food supplement composition usually comprises at least about 1 wt%, for example at least about 2 wt%, preferably at least about 3 wt% of alpha lipoic acid based on the total weight of the pet food supplement composition.

The pet food supplement composition typically comprises up to about 10 wt%, for example up to about 8 wt%, preferably up to about 6 wt% of alpha lipoic acid based on the total weight of the pet food supplement composition.

Usually, the pet food supplement composition comprises from about 1 wt% to about 10 wt%, for example from about 2 wt% to about 8 wt%, preferably from about 3 wt% to about 6 wt% of alpha lipoic acid based on the total weight of the pet food supplement composition.

For example, the pet food supplement composition may comprise about 3.3 wt% of alpha lipoic acid based on the total weight of the pet food supplement composition.

The pet food supplement composition comprises L-carnitine. Without wishing to be bound by theory, it is thought that L-carnitine increases basal metabolic rate, supporting weight loss and antioxidant activity associated with inflammatory excess weight.

The pet food supplement composition typically comprises at least about 2 wt%, for example at least about 4 wt%, preferably at least about 5 wt% of L-carnitine based on the total weight of the pet food supplement composition.

The pet food supplement composition usually comprises up to about 30 wt%, for example up to about 20 wt%, preferably up to about 12 wt% of L-carnitine based on the total weight of the pet food supplement composition.

Typically, the pet food supplement composition comprises from about 2 wt% to about 30 wt%, for example from about 4 wt% to about 20 wt%, preferably from about 5 wt% to about 12 wt% of L-carnitine based on the total weight of the pet food supplement composition.

For example, the pet food supplement composition may comprise about 6.7 wt% L-carnitine based on the total weight of the pet food supplement composition.

The pet food supplement composition usually comprises at least about 0.05 wt%, for example at least about 0.1 wt%, preferably at least about 0.2 wt% of the probiotic based on the total weight of the pet food supplement composition.

The pet food supplement composition typically comprises up to about 0.5 wt%, for example up to about 0.4 wt%, preferably up to about 0.3 wt% of the probiotic based on the total weight of the pet food supplement composition.

The pet food supplement composition usually comprises from about 0.05 wt% to about 0.5 wt%, for example from about 0.1 wt% to about 0.4 wt%, preferably from about 0.2 wt% to about 0.3 wt% of the probiotic based on the total weight of the pet food supplement composition.

Preferably, the probiotic is from the genus *enterococcus,* for example, *enterococcus faecium. Enterococcus faecium* is a gram-positive bacterium. Without wishing to be bound by theory, it is thought that the probiotic, e.g, *enterococcus faecium,* may contribute to weight loss via microbiome modulation, e.g., canine microbiome modulation.

For example, the pet food supplement composition may comprise from about 0.05 wt% to about 0.15 wt%, preferably from about 0.075 wt% to about 0.14 wt%, *enterococcus faecium* based on the total weight of the pet food supplement composition.

For example, the pet food supplement composition may comprise about 0.2 wt% *enterococcus faecium* based on the total weight of the pet food supplement composition.

The probiotic may be provided in an amount measured by the number of colony forming units. The colony forming unit, or CFU, is a measure of the number of viable microbial cells in a sample. For example, CFU may be measured from a CFU assay using the International Standard ISO 15214:1998 - Microbiology of food and animal feeding stuffs - Horizontal method for the enumeration of mesophilic lactic acid bacteria - Colony-count technique at 30 °C.

The *enterococcus faecium* may comprise from about 1 to about 2 billion CFU, for example, from about 1.25 to 1.75 billion CFU, e.g., from about 1.25 to 1.75 billion CFU. For example, the *enterococcus faecium* may comprise about 1.5 billion CFU.

Typically, the pet food supplement composition further comprises a source of flavour. The flavour may, for example, enhance the palatability profile of the pet food supplement composition, increasing the likelihood that the companion animal will consume the pet food supplement composition. This makes weight reduction more likely to occur. The source of flavour may be referred to as a palatant.

The source of flavour may comprise nutrients that are beneficial to the health and/or welfare of the companion animal. The source of flavour may, for example, comprise vitamins and/or minerals.

The pet food supplement composition usually comprises at least about 10 wt%, for example at least about 15 wt%, preferably at least about 20 wt% of the source of flavour based on the total weight of the pet food supplement composition.

Typically, the pet food supplement composition comprises up to about 45 wt%, for example up to about 40 wt%, preferably up to about 35 wt% of the source of flavour based on the total weight of the pet food supplement composition.

The pet food supplement composition usually comprises from about 10 wt% to about 45 wt%, for example from about 15 wt% to about 40 wt%, preferably from about 20 wt% to about 35 wt% of the source of flavour based on the total weight of the pet food supplement composition.

The pet food supplement composition typically comprises up to about 33.1 wt% of the source of flavour based on the total weight of the pet food supplement composition.

The source of flavour may comprise brewer's yeast powder, nutritional yeast, yeast containing products or mixtures thereof. Alternatively, the source of flavour may be a protein source such as vegetable protein, e.g., a vegetable protein selected from the group consisting of pea protein and soya protein, and mixtures thereof. The source of flavour may comprise (a) brewer's yeast powder, nutritional yeast, yeast containing products or mixtures thereof, and (b) a protein source such as vegetable protein, e.g., a vegetable protein selected from the group consisting of pea protein and soya protein, and mixtures thereof.

The source of flavour may additionally comprise coconut. For example, the source of flavour may comprise brewer's yeast and coconut.

Preferably, the source of flavour comprises a yeast. Preferably the yeast comprises brewer's yeast.

The source of flavour may comprise brewer's yeast and coconut.

For example, the pet food supplement composition may comprise from about 10 wt% to about 45 wt%, for example from about 15 wt% to about 40 wt%, preferably from about 20 wt% to about 35 wt% of the source of flavour based on the total weight of the pet food supplement composition, and the source of flavour comprises brewer's yeast.

The pet food supplement composition may comprise from about 10 wt% to about 45 wt%, for example from about 15 wt% to about 40 wt%, preferably from about 20 wt% to about 35 wt% of the source of flavour based on the total weight of the pet food supplement composition, and the source of flavour comprises brewer's yeast and coconut.

The pet food supplement composition usually comprises: at least about 20 wt%, for example at least about 30 wt%, preferably at least about 40 wt% of the source of resistant starch; at least about 2 wt%, for example at least about 4 wt%, preferably at least about 5 wt% of the source of beta glucan; at least about 0.05 wt%, for example at least about 0.1 wt%, preferably at least about 0.2 wt% of the probiotic; at least about 2 wt%, for example at least about 4 wt%, preferably at least about 5 wt% of L-carnitine; and at least about 1 wt%, for example at least about 2 wt%, preferably at least about 3 wt% of alpha lipoic acid, and optionally at least about 10 wt%, for example at least about 15 wt%, preferably at least about 20 wt% of the source of flavour, based on the total weight of the pet food supplement composition.

The pet food supplement composition usually comprises: up to about 90 wt%, for example up to about 70 wt%, preferably up to about 60 wt% of the source of resistant starch; up to about 30 wt%, for example up to about 20 wt%, preferably up to about 12 wt%, of the source of beta glucan; up to about 0.5 wt%, for example up to about 0.4 wt%, preferably up to about 0.3 wt% of the probiotic; up to about 30 wt%, for example up to about 20 wt%, preferably up to about 12 wt% of L-carnitine; and up to about 10 wt%, for example up to about 8 wt%, preferably up to about 6 wt% of alpha lipoic acid, and optionally up to about 45 wt%, for example up to about 40 wt%, preferably up to about 35 wt% of the source of flavour, based on the total weight of the pet food supplement composition.

For example, the pet food supplement composition may comprise: from about 20 wt% to about 90 wt%, for example from about 30 wt% to about 70 wt%, preferably from about 40 wt% to about 60 wt% of the source of resistant starch; from about 2 wt% to about 30 wt%, for example from about 4 wt% to about 20 wt%, preferably from about 5 wt% to about 12 wt% of the source of beta glucan; from about 0.05 wt% to about 0.5 wt%, for example from about 0.1 wt% to about 0.4 wt%, preferably from about 0.2 wt% to about 0.3 wt% of the probiotic; from about 2 wt% to about 30 wt%, for example from about 4 wt% to about 20 wt%, preferably from about 5 wt% to about 12 wt%, L-carnitine; and from about 1 wt% to about 10 wt%, for example from about 2 wt% to about 8 wt%, preferably from about 3 wt% to about 6 wt% of alpha lipoic acid, and optionally from about 10 wt% to about 45 wt%, for example from about 15 wt% to about 40 wt%, preferably from about 20 wt% to about 35 wt% of the source of flavour, based on the total weight of the pet food supplement composition.

The pet food supplement composition may comprise: green banana fruit pulp extract; oat beta glucan; rice bran; alpha lipoic acid; L-carnitine; *enterococcus faecium;* and brewer's yeast.

Preferably, the pet food supplement composition comprises: green banana fruit pulp extract; oat beta glucan; alpha lipoic acid; L-carnitine; *enterococcus faecium*; and a source of flavour comprising brewer's yeast.

More preferably, the pet food supplement composition comprises: green banana fruit pulp extract; oat beta glucan; alpha lipoic acid; L-carnitine; *enterococcus faecium*; and a source of flavour comprising brewer's yeast and coconut.

The pet food supplement composition may comprise: at least about 20 wt%, for example at least about 30 wt%, preferably at least about 40 wt%, green banana fruit pulp extract; at least about 2 wt%, for example at least about 4 wt%, preferably at least about 5 wt%, oat beta glucan; at least about 0.05 wt%, for example at least about 0.1 wt%, preferably at least about 0.2 wt%, *enterococcus faecium*; at least about 2 wt%, for example at least about 4 wt%, preferably at least about 5 wt% of L-carnitine; and at least about 1 wt%, for example at least about 2 wt%, preferably at least about 3 wt% of alpha lipoic acid, and at least about 10 wt%, for example at least about 15 wt%, preferably at least about 20 wt%, brewer's yeast, based on the total weight of the pet food supplement composition.

The pet food supplement composition may comprise: up to about 90 wt%, for example up to about 70 wt%, preferably up to about 60 wt%, green banana fruit pulp extract; up to about 30 wt%, for example up to about 20 wt%, preferably up to about 12 wt%, oat beta glucan; up to about 0.5 wt%, for example up to about 0.4 wt%, preferably up to about 0.3 wt%, *enterococcus faecium*; up to about 30 wt%, for example up to about 20 wt%, preferably up to about 12 wt% of L-carnitine; and up to about 10 wt%, for example up to about 8 wt%, preferably up to about 6 wt% of alpha lipoic acid, and up to about 45 wt%, for example up to about 40 wt%, preferably up to about 35 wt%, a source of flavour comprising brewer's yeast, based on the total weight of the pet food supplement composition.

For example, the pet food supplement composition may comprise: from about 20 wt% to about 90 wt%, for example from about 30 wt% to about 70 wt%, preferably from about 40 wt% to about 60 wt% green banana fruit pulp extract; from about 2 wt% to about 30 wt%, for example from about 4 wt% to about 20 wt%, preferably from about 5 wt% to about 12 wt% oat beta glucan; from about 0.05 wt% to about 0.5 wt%, for example from about 0.1 wt% to about 0.4 wt%, preferably from about 0.2 wt% to about 0.3 wt%, *enterococcus faecium*; from about 2 wt% to about 30 wt%, for example from about 4 wt% to about 20 wt%, preferably from about 5 wt% to about 12 wt%, L-carnitine; and from about 1 wt% to about 10 wt%, for example from about 2 wt% to about 8 wt%, preferably from about 3 wt% to about 6 wt%, alpha lipoic acid, and from about 10 wt% to about 45 wt%, for example from about 15 wt% to about 40 wt%, preferably from about 20 wt% to about 35 wt%, a source of flavour comprising brewer's yeast, based on the total weight of the pet food supplement composition.

The pet food supplement composition may comprise: about 50 wt% green banana fruit pulp extract; about 6.7 wt% oat beta glucan; about 3.3 wt% alpha lipoic acid; about 6.7 wt% L-carnitine; about 0.2 wt% *enterococcus faecium*; and about 33.1 wt% a source of flavour comprising brewer's yeast .

### Uses of the pet food supplement composition

The present inventors have found that the pet food supplement composition is particularly effective in reducing and/or controlling the weight of a companion animal, particularly a dog. The reduction in weight observed when the companion animal consumes the pet food supplement composition may lead to additional health benefits, such as a reduced risk of heart disease.

Advantageously, the pet food supplement composition provides improvements in the microbiome of the companion animal and/or increases the metabolism of the companion animal and/or acts as a appetite suppressor.

Surprisingly, the improvement in health observed, particularly the weight loss observed when a companion animal receives the pet food supplement composition, is greater than would have been anticipated when considering the health benefits of the ingredients being administered individually.

The pet food supplement composition may therefore be used in the enhancement of pet health.

The invention provides a pet food supplement composition for use in the enhancement of pet health, wherein: the pet food supplement composition is as defined herein; and the enhancement of pet health is the treatment of obesity.

One or more of the following may be observed following administration of the pet food supplement composition: lowering of body weight; improved body condition score; and/or improvement in other biomarkers of health such as blood lipid levels and c-reactive protein (indicator of inflammation).

The pet food supplement composition may alternatively be useful for non-therapeutic uses, including the promotion of weight loss, regulation of body weight and/or maintaining a healthy weight in a companion animal that is not suffering from obesity.

The invention therefore also provides use of a pet food supplement composition for promoting weight loss or maintaining a healthy weight, wherein: the pet food supplement composition is as defined herein.

### Administration of the pet food supplement composition

The pet food supplement composition may be formulated to be administered with food, i.e., for oral administration. Thus, for example, the companion animal (e.g., dog) may consume the pet food supplement composition with a meal. The inventors have found that providing the pet food supplement composition with food provides optimum results.

Also provided by the invention therefore is the pet food supplement composition for use as described herein or the use of a pet food supplement composition defined herein, wherein the pet food supplement composition is provided with food.

Preferably the pet food supplement composition forms part of a healthy, balanced diet, e.g., a diet in which calorie and fat levels are carefully controlled. However, the pet food supplement composition may also be beneficial for use when the companion animal's diet would not be considered a healthy or balanced diet, e.g., when the diet contains higher levels of fat and/or more calories than recommended for the companion animal.

Other options for controlling and/or reducing weight include the use of diets, i.e., the reduction of food quantities and/or restriction of food items consumed by the companion animal. The inventors have found that providing the pet food supplement composition for administration with food improves compliance when compared to the use of a diet, e.g., when compared to the use of a diet having the same overall calorie count and fat content as provided by the pet food supplement composition and food the pet food supplement composition is provided with. Improved compliance results in improved health benefits, such as increased weight loss and/or weight loss sustained over a longer period of time.

The combination of ingredients in the pet food supplement composition of the invention provides unexpected synergistic benefits. Thus, the combination of ingredients provides benefits over and above the benefits that would be expected based on the ingredients provided individually.

Without wishing to be bound by theory, at least some of the advantages provided by the present invention are thought to arise due to the beneficial effect provided when the pet food supplement composition is mixed with fat consumed by the companion animal.

The intestines of the companion animal absorb fat from the companion animal's diet. The pet food supplement composition may coat fat consumed by the companion animal preventing fat absorption or reducing the level of fat absorption into the body of the companion animal. For example, fat coated with the pet food supplement composition may not pass through the intestinal lumen or may pass through the intestinal lumen in lower quantities. The pet food supplement composition may, therefore, provide a coating that blocks or reduces absorption of fat.

The inclusion of L-carnitine, together with the other ingredients in the pet food composition, is thought to boost fat metabolism. Boosting metabolism, e.g., at a cellular level, may provide additional unexpected but beneficial changes in the gut microbiome of the companion animal (e.g., the presence of more helpful bacteria) that further improves weight loss and/or lowers fat absorption from the diet. The increase in metabolic rate and/or fat metabolism provided by L-carnitine may additionally promote weight loss due to an increased resting calorie burn. The presence of L-carnitine may, therefore, provide an unexpected acceleration of the effects provided by other ingredients in the pet food supplement composition.

### Dosing of the pet food supplement composition

Typically, the pet food supplement composition is administered at least once daily, e.g., at least in the morning each day or at least in the evening each day. The pet food composition may, for example, be provided with breakfast or with an evening meal.

For example, the pet food supplement composition may be administered once, twice or three times daily. Administration twice daily, for example, may mean the pet food supplement composition is provided with breakfast and with an evening meal. Administration three times daily, for example, may mean the pet food supplement composition is provided with breakfast, with lunch and with an evening meal.

Not all companion animals (e.g., dogs) follow the same feeding pattern. It may not be necessary for the companion animal to consume the pet food supplement with every meal. For example, the pet food supplement composition may be consumed with breakfast but not with lunch and/or with dinner. If, for example, the companion animal (e.g., dog) only has one meal a day then the pet food supplement composition is typically consumed with all meals, i.e., once daily. The amount of the pet food supplement composition provided with a meal may need to be adjusted to take into account the feeding pattern of the companion animal.

As the skilled person will appreciate, the amount of the pet food supplement composition provided will vary depending on the companion animal. For dogs, the amount of the pet food supplement composition provided may vary depending on factors such as the breed, age, sex, and/or weight of the dog.

The pet food supplement composition for use as described herein or the use of a pet food supplement composition as described herein may be administered in a dose of at least about 0.5 g, e.g., at least about 0.75 g, for example at least about 1 g.

Optionally, the pet food supplement composition for use as described herein or the use of a pet food supplement composition as described herein may be administered in a dose of at least about 0.5 g, e.g., at least about 0.75 g, for example at least about 1 g, wherein the dose is provided at least once daily, e.g., once, twice or three times daily. For example, the pet food supplement composition for use as described herein or the use of a pet food supplement composition as described herein may be administered in a dose of at least about 0.5 g, e.g., at least about 0.75 g, for example at least about 1 g, wherein the dose is provided three times daily.

The pet food supplement composition for use as described herein or the use of a pet food supplement composition as described here may be administered in a dose of up to about 5.0 g, e.g., up to about 4.0 g, for example up to about 3.0 g.

The pet food supplement composition for use as described herein or the use of a pet food supplement composition as described herein may be administered in a dose of up to about 5.0 g, e.g., up to about 4.0 g, for example up to about 3.0 g, wherein the dose is provided at least once daily, e.g., once, twice or three times daily.

The pet food supplement composition for use as described herein or the use of a pet food supplement composition as described here may be administered in a dose of from about 0.5 g to about 5 g, e.g., from about 0.75 g to about 4 g, for example from about 1 g to about 3 g.

The pet food supplement composition for use as described herein or the use of a pet food supplement composition as described herein may be administered in a dose of from about 0.5 g to about 5 g, e.g., from about 0.75 g to about 4 g, for example from about 1 g to about 3 g, wherein the dose is provided at least once daily, e.g., once, twice or three times daily.

The amount of pet food supplement composition provided may be about 1.5 g, i.e., about 1.5 g provided at least one daily (e.g., once, twice or three times daily).

### Producing the pet food supplement composition

Typically, the pet food supplement composition is provided as a powder, optionally wherein the pet food supplement composition is provided as an intimate mixture of the individual ingredients.

The term "intimate mixture" as used herein refers to a mixture of ingredients in which the individual components are thoroughly mixed and/or randomly distributed throughout the mixture. This is advantageous because it avoids portions of the pet food supplement composition being deficient in one or more of the individual components. Providing the pet food supplement composition as an intimate mixture therefore helps to ensure that the companion animal is provided with each of the individual components, at an appropriate weight ratio, each time the pet food supplement composition is administered.

The present invention provides a process for producing the pet food supplement composition described herein, which process comprises (a) providing the source of resistant starch; the source of beta glucan; the probiotic; the L-carnitine; and the alpha lipoic acid; and optionally the source of flavour; and (b) mixing together the source of resistant starch; the source of beta glucan; the probiotic; the L-carnitine; and the alpha lipoic acid; and optionally the source of flavour. Preferably the step of mixing provides an intimate mixture of the individual components.

Also provided is a process for producing the pet food supplement composition described herein, which process comprises (a) providing the alpha lipoic acid and the probiotic; (b) mixing together the alpha lipoic acid and the probiotic; (c) additionally providing the source of resistant starch; the source of beta glucan; and the L-carnitine; and optionally the source of flavour; and (d) mixing together the source of resistant starch; the source of beta glucan; and the L-carnitine; and optionally the source of flavour, with the alpha lipoic acid and *enterococcus faecium* mixture provided by step (b). When relatively small amounts of the alpha lipoic acid and the probiotic are present in the pet food supplement composition, premixing the alpha lipoic acid powder and the probiotic may advantageously improve mixing efficiency.

The providing step(s) typically require the individual ingredients to be combined in weight ratios that match the weight ratio of the individual ingredients in the pet food supplement composition.

Mixing may, for example, take place in a mixing vessel, e.g., comprising mixing blades. The inventors have found that mixing efficiency may be increased by avoiding placing individual ingredients into one location within the mixing vessel and/or by avoiding placing individual ingredients on to the mixing blades prior to mixing. An increase in mixing efficiency reduces the risk of areas of mixture being devoid of, or comprising a reduced amount of, a particular component of the pet food supplement composition. Areas devoid of, or comprising a reduced amount of, a particular component may be referred to as "dead spots".

The invention also provides an extruded product, such as a chew, comprising the pet food supplement composition described herein.

The present invention therefore provides an extruded product comprising a pet food supplement composition, wherein the pet food supplement composition is as defined herein, optionally wherein the extruded product is an extruded soft chew, e.g., a cold extruded soft chew.

Methods of extrusion include those known to the person skilled in the art, e.g., cold extrusion and hot extrusion. Extrusion may be performed with a high or a low moisture content.

As the skilled person will appreciate, to provide a soft chew, the mixture of ingredients provided by the process defined herein for producing the pet food supplement composition should be added to oil and aqueous components. Typically the dry ingredient mixture is first added to an oil component, e.g., refined vegetable oil (sunflower). This mixture is then usually combined with an aqueous mixture. The aqueous mixture may comprise deionised water. The aqueous mixture may additionally comprise sorbic acid powder and/or glycerine. Typically, the aqueous mixture comprises deionised water, sorbic acid powder and glycerine.

As an example, the amount of deionised water, sorbic acid powder and glycerine added, based on the total weight of the mixture including the pet food supplement composition, oil component and aqueous mixture, may be: from about 2 wt% to about 4 wt% deionised water; from about 0.25 wt% to about 0.75 wt% sorbic acid; and from about 12 wt% to about 25 wt%

In a further alternative, the pet food supplement composition may be provided as a pressed product (e.g., cold pressed), a freeze dried product, a steam baked product, or an oven baked product.

The skilled person will appreciate that, when preparing the pet food supplement composition care should be taken to avoid the use of conditions (e.g., temperatures) that may have a negative effect on one or more of the individual components. For example, conditions (e.g., temperatures) that may denature ingredients such as the probiotic and/or the source of flavour and/or damage heat-sensitive nutrients should be avoided.

### Examples

### Ingredients

The ingredients set out below were used in the examples that follow.
- green banana fruit pulp extract - dried banana pulp
- oat beta glucan
- rice bran
- alpha lipoic acid (thioctic acid)
- L-carnitine - L-carnitine base vegan
- *enterococcus faecium - Enterococcus faecium NCIMB 10415 (DSM 10663)*
- brewer's yeast
- B25017 Coral 101170 - a yeast based flavour enhancer comprising sodium chloride, sugars, refined vegetable oil
- Coconut - coconut flour fine desiccated (cocos nucifera) NHT

### Example 1

The following components were combined to provide a pet food supplement composition:
- 750 mg green banana fruit pulp;
- 100 mg oat beta glucan;
- 50 mg alpha lipoic acid powder;
- 100 mg L-carnitine; and
- 0.2 mg *enterococcus faecium.*

### Pet food supplement composition in powder form

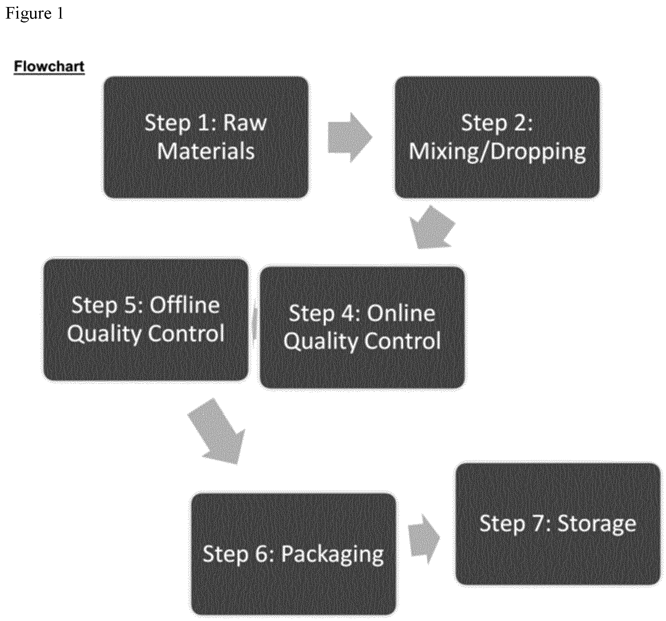

A powder composition comprising the pet food supplement composition was prepared using the Standard Operating Procedure (SOP) set out in Table 1 and illustrated in figure 1. The ratio of ingredients within the powder composition is the same as the ratio of ingredients in example 1 above. However, for manufacturing efficiency and to provide multiple portions of the pet food supplement composition of example 1, the powder composition weighed several kilograms. Once produced, the batch was divided up into portions, or doses, of the pet food supplement composition.

**Table 1**

| **Flowchart Step** | **Description** |
|---|---|
| Step 1: Raw Materials | Ensure all raw materials meet quality standards before use. |
| | Follow respective formulas and manufacturing instructions to |
| | ensure correct mixing quantities and methods are adhered to. |
| | Weigh out correct quantities of raw materials to be mixed. |
| Step 2: Mixing/Dropping | Combine raw materials according to specific, respective formulas manufacturing instructions. Transfer from mixer to tote. |
| Step 3: Online Quality Control | Follow online quality control procedures (e.g. check weights). |
| Step 4: Offline Quality Control | Perform offline quality checks on finished products. |
| Step 5: Packaging | Package the finished products. |
| Step 6: Storage | Store packaged products in the designated area. |

The alpha lipoic acid powder and *enterococcus faecium* were weighed out into the same mixing vessel, the lid of the mixing vessel was closed and the alpha lipoic acid powder and *enterococcus faecium* were mixed together to provide a homogeneous mixture. Mixing took approximately 1 minute. This premixing was found to assist mixing efficiency.

The green banana fruit pulp, oat beta glucan, and L-carnitine were weighed out and added to the same mixing vessel as the pre-mixed alpha lipoic acid powder and *enterococcus faecium.* The lid of the mixing vessel was closed. All of the ingredients were then mixed to provide a homogeneous mixture. Mixing took approximately 2 minutes. A high refiner / chopper was used for the final 30 seconds of mixing. Once mixing was complete, the mixing vessel was opened and the powder was checked for "dead spots" before the mixture was emptied from the mixing vessel.

### Soft chew comprising the pet food supplement composition

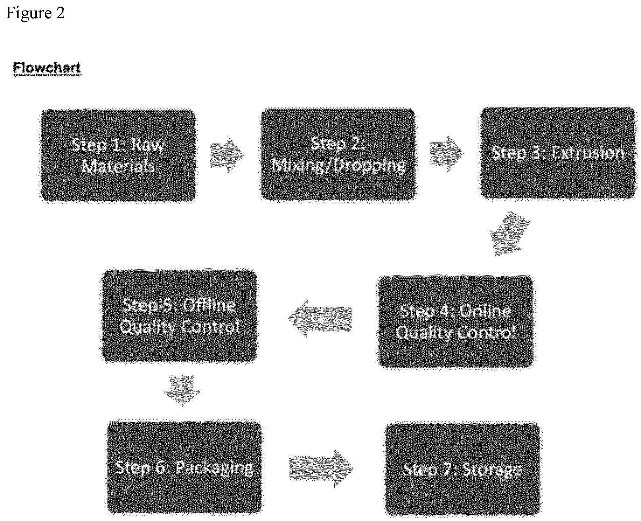

A soft chew comprising the pet food supplement composition of example 1 was prepared using the Standard Operating Procedure (SOP) set out in Table 2 and illustrated in figure 2.

**Table 2**

| **Flowchart Step** | **Description** |
|---|---|
| Step 1: Raw Materials | Ensure all raw materials meet quality standards before use. |
| | Follow respective formulas and manufacturing instructions to ensure correct mixing quantities and methods are adhered to. |
| | Weigh out correct quantities of raw materials to be mixed. |
| Step 2: Mixing/Dropping | Combine raw materials according to specific, respective formulas manufacturing instructions. Transfer from mixer to tote. |
| Step 3: Extrusion | Transfer material to extruder and extrude mixed material into chews using the cutter. Cut to respective size as dictated by |
| Step 4: Online Quality Control | Follow online quality control procedures (e.g. check chew weights). |
| Step 5: Offline Quality Control | Perform offline quality checks on finished products. |
| Step 6: Packaging | Package the finished products. |
| Step 7: Storage | Store packaged products in the designated area. |

A powder composition was prepared as set out above. Refined vegetable oil (sunflower) was added to the powder composition. The lid of the mixing vessel was closed and the ingredients mixed for approximately 2 minutes. A high refiner / chopper was used for the final 30 seconds of mixing.

**In** a separate mixer, deionised water and sorbic acid powder (food grade) were mixed together using a whisk for approximately 2 minutes. Glycerine was then added and the ingredients mixed for a further 1 minute.

All of the ingredients (the refined vegetable oil (sunflower) combined with the powder composition, and the mix of deionised water, sorbic acid powder (food grade) and glycerine) were mixed together, slowly, in the mixing vessel, over the course of several minutes. To avoid over mixing, the high refiner / chopper was not used for more than 2 minutes. The mixture was then left to rest for 1 hour, inside the mixing vessel. After removal from the mixing vessel, the product was extruded and cut into chunks.

### Example 2

The following components were combined to provide a pet food supplement composition:
- 750 mg green banana fruit pulp;
- 100 mg oat beta glucan;
- 50 mg alpha lipoic acid powder;
- 100 mg L-carnitine;
- 0.2 mg *enterococcus faecium*;
- 392 mg brewer's yeast; and
- 105 mg B25017 Coral 101170.

### Example 3

The following components were combined to provide a pet food supplement composition:
- 750 mg rice bran;
- 100 mg oat beta glucan;
- 50 mg alpha lipoic acid powder;
- 100 mg L-carnitine base vegan; and
- 0.2 mg *enterococcus faecium.*

### Example 4

The following components were combined to provide a pet food supplement composition:
- 375 mg green banana fruit pulp;
- 375 mg rice bran;
- 100 mg oat beta glucan;
- 50 mg alpha lipoic acid powder;
- 100 mg L-carnitine; and
- 0.2 mg *enterococcus faecium.*

### Example 5

The following components were combined to provide a pet food supplement composition:
- 500 mg green banana fruit pulp;
- 500 mg oat beta glucan;
- 447 mg rice bran;
- 50 mg alpha lipoic acid powder;
- 500 mg L-carnitine base vegan;
- 3 mg *enterococcus faecium;* and
- 500 mg Brewer's yeast.

### Example 6

The following components were combined to provide a pet food supplement composition:
- 500 mg green banana fruit pulp;
- 500 mg oat beta glucan;
- 50 mg alpha lipoic acid powder;
- 500 mg L-carnitine base vegan;
- 3 mg *enterococcus faecium;* and
- 500 mg Brewer's yeast.

### Example 7

The following components were combined to provide a pet food supplement composition:
- 500 mg green banana fruit pulp;
- 447 mg rice bran;
- 50 mg alpha lipoic acid powder;
- 500 mg L-carnitine base vegan;
- 3 mg *enterococcus faecium*; and
- 500 mg Brewer's yeast.

### Example 8

The following components were combined to provide a pet food supplement composition:
- 500 mg oat beta glucan;
- 447 mg rice bran;
- 50 mg alpha lipoic acid powder;
- 500 mg L-carnitine base vegan;
- 3 mg *enterococcus faecium*; and
- 500 mg Brewer's yeast.

### Example 9

The following components were combined to provide a pet food supplement composition:
- 500 mg green banana fruit pulp;
- 500 mg oat beta glucan;
- 447 mg rice bran;
- 50 mg alpha lipoic acid powder;
- 500 mg L-carnitine base vegan;
- 3 mg *enterococcus faecium*; and
- 500 mg Brewer's yeast.

### Example 10

Sample size: 30 dogs
Dog type: Mixed small breeds, age range 10 months to 16 years old
Environment: Kennels / owner kept dogs
Prior diet: Wet food / dry food/ raw / fresh

### Test period: 6 Weeks

The pet food supplement composition of example 1 is provided to 30 dogs as a supplement to their previous food diet. Each dog is weighed and body condition scored immediately prior to administration of the pet food supplement composition and their weight is assessed against weight recommendations for their breed, sex and age. The weight and body condition score of each dog is then monitored regularly over the test period. Other variables such as begging behaviours for food and satiety are assessed via a client and/or dog carer periodically using a Likert type survey.

The experiment is repeated with the pet food supplement composition of examples 2-9.

### Example 11

A blinded, cross-over study was carried out to establish whether a composition containing resistant-starch, beta-glucan, alpha lipoic acid, L-carnitine and a probiotic would cause weight loss in overweight/obese dogs. The null hypothesis was that this resistant-starch containing supplement blend administered for a 30 day period would not cause significant weight loss in overweight/obese dogs.

The pet food supplement composition tested was:
- 750 mg green banana fruit pulp;
- 100 mg oat beta glucan (70%);
- 50 mg alpha lipoic acid powder;
- 100 mg L-carnitine;
- *enterococcus faecium*;
- 13.5 mg coconut;
- 378.5 mg brewer's yeast; and
- 105 mg B25017 Coral 101170.

The placebo composition was:
- 36 mg coconut;
- 1359 mg brewer's yeast; and
- 105 mg B25017 Coral 101170

### Materials and methods

27 home-based dogs were recruited into the study. All dogs underwent a clinical examination by a veterinarian before inclusion in the study. The veterinarian oversaw the dogs health throughout the study and was responsible for weighing and assessing body condition scores on days 0, 30 and 65.

Dog ages ranged from 3 years to 10 years (Mean (Average) 6.71, Median 6, Range 7, Mode 6, appeared 6 times, Geometric Mean 6.38, Largest 10, Smallest 3). There were 11 males (55%), 9 females (45%).

Initial Body weights: Mean 16.18kg, Median 13.9kg, Mode 9.1kg, Range 32.7kg, Minimum 5.7kg, Maximum 38.4kg. (See Table 3)

Initial Body Condition Scores: Mean 7.93, Median 8, Mode 8, Range 2, Minimum 7, Maximum 9. So, 13 dogs were clinically obese having a BCS of 8/9, the other 8 dogs were overweight BCS 7-7.5 (See Table 4) (The BCS value is a Body Condition Score for dogs ranging from 1 to 9 (Association for Pet Obesity Prevention). The values may be summarised as follows: 1 emaciated, 2 very thin, 3, thin, 4 moderately thin, 5 ideal, 6 moderately above ideal, 7 overweight, 8 obesity, and 9 severe obesity.)

Dogs were living in their home environment, exercised and fed their usual ration throughout the study period. They were weighed on Day 0 then given the supplement containing active ingredients mixed into their regular ration for 30 days, followed by a 5 day wash-out period with no supplement. The dogs were then given the placebo for 30 days. The owners and veterinary surgeon overseeing the study were masked to the content of the powder supplements being administered.

Outcome measures were body weight change (objective) and body condition score assessment using a validated 9 point scoring system, BCS (subjective). The dogs were weighed and body condition score was assessed by a veterinarian on days 0, 30 and 65.

The study was conducted in accordance with ethical guidelines from the Royal College of Veterinary Surgeon on clinical research in practice (RCVS 2024).

### Results

6 dogs (22.22%) did not complete the study due to concurrent non-related medical problems (n=3) or refusal to eat the product (n=3). 21 dogs completed the study (77.78%).

### After 30 days on the supplement containing resistant-starch and other active ingredients:

Body weight changes: 17 dogs (80.95%) lost weight: Mean 0.63kg, Median 0.4kg, Mode 0.3kg, Range 1.7kg, Minimum 0.1kg Maximum 1.8kg. (Table 3)

The % body weight loss was: Mean (Average) 3.52, Median 3.3, Range 4.46, Mode All values appeared just once. Geometric Mean 3.26 Largest 5.76, Smallest 1.3. (Table 3). In one dog there was no change, and 3 dogs (14.29%) gained weight (Mean 0.2kg, Median 0.1kg, Mode 0.1kg Range 0.3kg Minimum 0.1kg, Maximum 0.4kg). (Table 3)

**Table 3 Body Weight Changes**

| **DOG** | **DAY 0** | **DAY 30** | **CHANGE** | **% CHANGE** | **DAY 65** | **CHANGE** | **% CHANGE** |
|---|---|---|---|---|---|---|---|
| 1. | 9.1 kg | 8.7 kg | -0.4 kg | - 4.4 % | 8.6 kg | -0.1 kg | - 1.16 % |
| 2. | 5.7 kg | 5.8 kg | +0.1 kg | +1.75 % | 5.5 kg | -0.3 kg | - 5.45 % |
| 3. | 15.3 kg | 15.1 kg | -0.2 kg | - 1.3 % | 14.2 kg | -0.9 kg | - 6.34 % |
| 4. | 8.3 kg | 8 kg | -0.3 kg | - 3.61 % | 7.9 kg | -0.1 kg | - 1.27 % |
| 5. | 10.2 kg | 9.9 kg | -0.3 kg | - 2.94 % | 9.7 kg | -0.2 kg | - 2.06 % |
| 6. | 8.4 kg | 8.8 kg | +0.4 kg | + 4.76 % | 7.8 kg | -1.0 kg | -12.82 % |
| 7. | 9.4 kg | 9.7 kg | -0.3 kg | - 3.19 % | 9 kg | -0.7 kg | - 7.78 % |
| 8. | 6.1 kg | 6 kg | -0.1 kg | - 1.64 % | 6 kg | 0 | 0 |
| 9. | 38.4 kg | 37.5 kg | -0.9 kg | - 2.34 % | 37.5 kg | 0 | 0 |
| 10. | 13.2 kg | 12.9 kg | -0.3 kg | - 2.27 % | 12.8 kg | -0.1 kg | - 0.78% |
| 11. | 20.9 kg | 21 kg | +0. 1 kg | +0.48 % | 20.4 kg | -0.6 kg | - 2.94 % |
| 12. | 9.1 kg | 8.8 kg | -0.3 kg | - 3.3 % | 8.6 kg | -0.2 kg | - 2.33 % |
| 13. | 22.6 kg | 21.7 kg | -0.9 kg | - 3.98 % | 21.3 kg | -0.4 kg | - 1.88 % |
| 14. | 38.1 kg | 36.3 kg | -1.8 kg | -4.93 % | 34.4 kg | -1.9 kg | - 5.52 % |
| 15. | 32.5 kg | 30.8 kg | -1.7 kg | - 5.23 % | 29 kg | -1.8 kg | - 6.2 % |
| 16. | 14.4 kg | 14.4 kg | 0 | 0 | 14.2 kg | -0.2 kg | - 1.55 % |
| 17. | 19.8 kg | 19.2 kg | -0.6 kg | - 3.03 % | 19.1 kg | -0.1 kg | - 0.52 % |
| 18. | 14.8 kg | 14 kg | -0.8 kg | - 5.4 % | 14.1 kg | -0.1 kg | - 0.71 % |
| 19. | 13.9 kg | 13.1 kg | -0.8 kg | - 5.76 % | 13 kg | -0.1 kg | - 0.77 % |
| 20. | 17.1 kg | 16.4 kg | -0.7 kg | -4.09 % | 16.2 kg | -0.2 kg | - 1.23 % |
| 21. | 12.4 kg | 12.1 kg | -0.3 kg | - 2.42 % | 12 kg | -0.1 kg | - 0.83 % |

BCS Changes: In 7 dogs there was no change in BCS , one dog gained BCS (0.5) and 13 dogs had a reduction in BCS Mean (Average) 0.74, Median 0.5, Range 1.4, Mode 0.5, appeared 6 times, Geometric Mean 0.63, Largest 1.5, Smallest 0.1. (Table 4)

**TABLE 4 Body Condition Score Changes**

| **DOGS** | **DAY 0** | **DAY 30** | **BCS POINT CHANGE** | **DAY 65** | **BCS POINT CHANGE** |
|---|---|---|---|---|---|
| 1 | 8 | 7 | -1 | 7 | 0 |
| 2 | 7.5 | 7.5 | 0 | 7 | -0.5 |
| 3 | 7 | 7 | 0 | 6 | -1 |
| 4 | 7.5 | 7 | -0.5 | 7 | 0 |
| 5 | 7.5 | 7 | -0.5 | 7 | 0 |
| 6 | 8 | 8.5 | +0.5 | 7 | -1.5 |
| 7 | 8 | 8 | 0 | 7 | -1 |
| 8 | 8 | 8 | 0 | 8 | 0 |
| 9 | 9 | 8.5 | -0.5 | 8.5 | 0 |
| 10 | 8.5 | 8 | -0.5 | 8 | 0 |
| 11 | 9 | 9 | 0 | 9 | 0 |
| 12 | 8 | 8 | 0 | 7 | -1 |
| 13 | 8.5 | 8 | -0.5 | 8 | 0 |
| 14 | 8 | 7 | -1 | 6 | -1 |
| 15 | 8 | 7 | -1 | 6.5 | -0.5 |
| 16 | 8 | 8 | 0 | 8 | 0 |
| 17 | 7.5 | 6.5 | -1 | 6 | -0.5 |
| 18 | 7.5 | 6.5 | -1 | 6.5 | 0 |
| 19 | 7.5 | 6 | -1.5 | 6 | 0 |
| 20 | 8 | 7 | -1 | 7 | 0 |
| 21 | 7.5 | 7 | -0.5 | 7 | 0 |

### After 30 days on the placebo

Body weight changes:19 dogs (90.48%) lost weight Mean (Average) 0.48 kg, Median 0.2kg Range1.8kg, Mode 0.1kg, appeared 7 times, Geometric Mean 0.28kg, Largest 1.9kg Smallest 0.1kg and in 2 dogs there was no change. (Table 3)

The % body weight loss were: Mean (Average) 3.27, Median 1.88 Range 12.3 Mode All values appeared just once. Geometric Mean 2.12, Largest 12.82, Smallest 0.52. (Table 3)

BCS changes: There was no change in BCS in 13 dogs (61.9%), and a fall in BCS in 8 dogs (Mean (Average) 0.875, Median 1, Range 1, Mode 1, appeared 4 times, Geometric Mean 0.81,Largest 1.5, Smallest 0.5. (Table 4)

The following were statistically significant between the active ingredient-containing powder and the placebo powder:
a. Body weight loss (p<0.001)
b. Body condition score reduction (p<0.001)

### Owner Questionnaire Feedback

21 owners (100%) returned completed questionnaires for 15 different breeds and one crossbreed. There were 2 each of Jack Russell terriers, Cocker spaniels, Staffordshire Bull terriers, and Miniature Bull terriers. There were single dogs from the following breeds Australian terrier, Bichon Frise, Chihuahua, Chinese crested, Danish Swedish Farm dog, Finish Lapponian, Icelandic Sheepdog, Japanese spitz, Nova Scotia Duck Tolling Retriever, Portuguese Water Dog, and a Russian toy.

18 dogs (85.7%) were fed twice daily, 2 were fed free access (ad lib) and one dog was fed once daily on the active ingredient, then twice daily whilst on the placebo.

14 dogs (66.7%) ate their food with the active ingredient supplement normally, 2 dogs ate the food but slowly, whereas 3 dogs ate it enthusiastically. One dog refused to eat it until a broth was added. 16 dogs (76%) ate their food with placebo normally, 2 ate it enthusiastically, and 2 ate it slowly. One owner completed this question incorrectly.

Whilst on the active ingredient supplement one dog did no exercise, 4 dogs (19%) exercised for up to 1 hour per day, 11 dogs (52%) exercised for over an hour per day and 4 dogs (19%) exercised for over 2 hours per day. One owner incorrectly answered this question. Whilst on the placebo 12 dogs (57%) were exercised for over 1 hour per day, 5 (23.8%) for up to 1 hour per day and 4 (19%)for over 2 hours per day.

20 dogs (95%) had normal faeces on the active ingredient, one dog had soft but formed stools. One dog had soft faeces before the trial which resolved on the active ingredient. All 21 dogs had normal faeces whilst on the placebo.

On the active ingredient-containing supplement 10 dogs (47.6z%) showed no change in appetite, 9 dogs (43%) appeared to be less hungry and 2 dogs were more hungry, one owner thought this was because the dog liked the supplement. On the placebo 16 (76%) dogs showed no change in appetite and 5 (23.8%) seemed less hungry.

Whilst on the active ingredient 19 dogs (90.5%) ate all of their food, 2 ate most of it, and on the placebo 18 dogs (85.7%) ate all the food and 3 dogs most of it.

20 owners (95%) found the active ingredient very easy to administer, and 19 (90.5%) found the placebo easy to administer. One owner had to adjust the feeding protocol for the active ingredient and 2 for the placebo.

The following observations were made when considering the results of the study: dogs lost weight at a healthy rate on supplement; clinically assed body fat reduced on supplement; dogs seemed less hungry on the supplement (begged less for food); some dogs seemed to enjoy the food even more when the supplement powder was mixed in suggesting it could even be a palatability enhancer for diets; adverse effects were incidental/not significant; several unexpected benefits were noted by participants (e.g., positive behavioural improvements) ; and several study participants were keen to buy the product post-trial proving commercial need (and possible lack of viable alternatives).

The results confirm the efficacy of the composition at reducing body weight and body condition scores in overweight/obese dogs, compared to placebo.

### EMBODIMENTS

The invention may also be understood by reference to the following numbered embodiments.
1. A pet food supplement composition comprising:
   a. a source of resistant starch;
   b. a source of beta glucan;
   c. a probiotic;
   d. L-carnitine; and
   e. alpha lipoic acid;
   wherein the source of resistant starch is a source of resistant starch derived from oat, rice, grains, legumes, fruit, raw potato starch, or high corn resistant starch, or combinations thereof.
2. The pet food supplement composition according to embodiment 1, comprising from about 20 wt% to about 90 wt% of the source of resistant starch based on the total weight of the pet food supplement composition.
3. The pet food supplement composition according to embodiment 1 or embodiment 2, wherein the source of resistant starch is green banana fruit pulp extract.
4. The pet food supplement composition according to any one of embodiments 1 to 3, comprising from about 2 wt% to about 30 wt% of the source of beta glucan based on the total weight of the pet food supplement composition.
5. The pet food supplement composition according to any one of embodiments 1 to 4, wherein the source of beta glucan is oat beta glucan.
6. The pet food supplement composition according to any one of embodiments 1 to 5, comprising from about 1 wt% to about 10 wt% of the alpha lipoic acid based on the total weight of the pet food supplement composition.
7. The pet food supplement composition according to any one of embodiments 1 to 6, comprising from about 2 wt% to about 30 wt% of the L-carnitine based on the total weight of the pet food supplement composition.
8. The pet food supplement composition according to any one of embodiments 1 to 7, comprising from about 0.05 wt% to about 0.5 wt% of the probiotic based on the total weight of the pet food supplement composition, wherein the probiotic is *enterococcus faecium.*
9. The pet food supplement composition according to any one of embodiments 1 to 8, wherein the pet food supplement composition further comprises a source of flavour.
10. The pet food supplement composition according to embodiment 9, comprising from about 10 wt% to about 45 wt% of the source of flavour based on the total weight of the pet food supplement composition, wherein the source of flavour comprises brewer's yeast.
11. The pet food supplement composition according to any one of embodiments 1 to 10, comprising:
   about 50 wt% green banana fruit pulp extract;
   about 6.7 wt% oat beta glucan;
   about 3.3 wt% alpha lipoic acid;
   about 6.7 wt% L-carnitine;
   about 0.2 wt% *enterococcus faecium*; and
   about 33.1 wt% a source of flavour comprising brewer's yeast.
12. A pet food supplement composition for use in the enhancement of pet health, wherein: the pet food supplement composition is as defined in any one of embodiments 1 to 11; and the enhancement of pet health is the treatment of obesity.
13. Use of a pet food supplement composition for promoting weight loss or maintaining a healthy weight, wherein the pet food supplement composition is as defined in any one of embodiments 1 to 11.
14. The pet food supplement composition for use according to embodiment 12 or the use of a pet food supplement composition according to embodiment 13, wherein the pet food supplement composition is provided with food.
15. The pet food supplement composition for use according to embodiment 12 or embodiment 14, or the use of a pet food supplement composition according to embodiment 13 or embodiment 14, wherein about 1.5 g of the pet food supplement composition is provided at least once daily.

## Claims

1. A pet food supplement composition comprising:
a. a source of resistant starch comprising green banana;
b. a source of beta glucan;
c. a probiotic;
d. L-carnitine; and
e. alpha lipoic acid.

2. The pet food supplement composition according to claim 1, comprising from about 20 wt% to about 90 wt% of the source of resistant starch based on the total weight of the pet food supplement composition.

3. The pet food supplement composition according to claim 1 or claim 2, wherein the source of resistant starch is green banana fruit pulp extract.

4. The pet food supplement composition according to any one of claims 1 to 3, comprising from about 2 wt% to about 30 wt% of the source of beta glucan based on the total weight of the pet food supplement composition.

5. The pet food supplement composition according to any one of claims 1 to 4, wherein the source of beta glucan is oat beta glucan.

6. The pet food supplement composition according to any one of claims 1 to 5, comprising from about 1 wt% to about 10 wt% of the alpha lipoic acid based on the total weight of the pet food supplement composition.

7. The pet food supplement composition according to any one of claims 1 to 6, comprising from about 2 wt% to about 30 wt% of the L-carnitine based on the total weight of the pet food supplement composition.

8. The pet food supplement composition according to any one of claims 1 to 7, comprising from about 0.05 wt% to about 0.5 wt% of the probiotic based on the total weight of the pet food supplement composition, wherein the probiotic is *enterococcus faecium.*

9. The pet food supplement composition according to any one of claims 1 to 8, wherein the pet food supplement composition further comprises a source of flavour, optionally wherein the pet food supplement composition comprises from about 10 wt% to about 45 wt% of the source of flavour based on the total weight of the pet food supplement composition, wherein the source of flavour comprises brewer's yeast.

10. The pet food supplement composition according to any one of claims 1 to 9, wherein the pet food supplement composition comprises: green banana fruit pulp extract; oat beta glucan; alpha lipoic acid; L-carnitine; *enterococcus faecium*; and a source of flavour comprising brewer's yeast.

11. The pet food supplement composition according to any one of claims 1 to 10, comprising:
about 50 wt% green banana fruit pulp extract;
about 6.7 wt% oat beta glucan;
about 3.3 wt% alpha lipoic acid;
about 6.7 wt% L-carnitine;
about 0.2 wt% *enterococcus faecium*; and
about 33.1 wt% a source of flavour comprising brewer's yeast.

12. A pet food supplement composition for use in the enhancement of pet health, wherein: the pet food supplement composition is as defined in any one of claims 1 to 11; and the enhancement of pet health is the treatment of obesity.

13. Use of a pet food supplement composition for promoting weight loss or maintaining a healthy weight, wherein the pet food supplement composition is as defined in any one of claims 1 to 11.

14. The pet food supplement composition for use according to claim 12 or the use of a pet food supplement composition according to claim 13, wherein the pet food supplement composition is provided with food.

15. The pet food supplement composition for use according to claim 12 or claim 14, or the use of a pet food supplement composition according to claim 13 or claim 14, wherein about 1.5 g of the pet food supplement composition is provided at least once daily.
